# EUROPÄISCHE PATENTSCHRIFT

(11) **EP 1 034 480 B1**
(45) Veröffentlichungstag und Bekanntmachung des Hinweises auf die Patenterteilung: **07.12.2005**
(21) Anmeldenummer: 98916931.3
(22) Anmeldetag: 12.03.1998
(51) Int. Cl.: G06F 13/00

(54) **EINRICHTUNG ZUR ZENTRALEN ÜBERWACHUNG UND/ODER STEUERUNG WENIGSTENS EINES GERÄTES**
ARRANGEMENT FOR THE CENTRAL MONITORING AND/OR CONTROL OF AT LEAST ONE APPARATUS
INSTALLATION DE SURVEILLANCE ET/OU DE COMMANDE CENTRALE D'AU MOINS UN APPAREIL

(30) Priorität: 12.03.1997 DE 19710191; 22.07.1997 DE 19733005
(43) Veröffentlichungstag der Anmeldung: 13.09.2000
(73) Patentinhaber: Storz Endoskop GmbH, 8200 Schaffhausen (CH)
(72) Erfinder: CHATENEVER, David, Santa Barbara, CA 93105 (US); IRION, Klaus, D-78576 Emmingen-Liptingen (DE); NOVAK, Pavel, CH-8207 Schaffhausen (CH); HILZINGER, Hans-Uwe, D-78532 Tuttlingen (DE)
(74) Vertreter: Meissner, Bolte & Partner
(86) Internationale Anmeldenummer: PCT/EP1998/001445
(87) Internationale Veröffentlichungsnummer: WO 1998/040822

(56) Entgegenhaltungen:
- EP-A- 0 355 042
- EP-A- 0 567 354
- WO-A-94/23375
- US-A- 4 688 168
- US-A- 4 774 568
- US-A- 4 870 704

## Beschreibung

### Technisches Gebiet

Die Erfindung bezieht sich auf eine Einrichtung zur zentralen Überwachung und/oder Steuerung wenigstens eines Gerätes für die Endoskopie und insbesondere für die minimal-invasive Chirurgie gemäß dem Oberbegriff des Patentanspruchs 1.

### Stand der Technik

Einrichtungen zur zentralen Überwachung und/oder Steuerung von endoskopischen Geräten sind beispielsweise aus der EP 0 319 762 A1 oder der prioritätsgleichen DE 37 41 833 C1, der US-PS 5 627 584, der WO 95/22363, der JP-A 62-46182 oder dem DE-U1 92 18 373 bekannt. Auf diese Druckschriften wird im übrigen zur Erläuterung aller hier nicht näher beschrieben Einzelheiten ausdrücklich Bezug genommen. Ein selbstkonfigurierender Bus ist in EP-A-0 355 042 beschrieben.

Die bekannten Einrichtungen, wie sie im Oberbegriff des Patentanspruchs 1 voraussetzt werden, können beispielsweise zur Steuerung einer oder mehrerer Insufflationsvorrichtungen, von Pumpen, einer Beleuchtungseinrichtung für das Bildfeld eines Endoskops, eines HF-Instruments zum Schneiden oder Koagulieren oder eines Lasers eingesetzt werden.

Bei den aus den genannten Druckschriften bekannten Einrichtungen ist das zu steuernde Gerät über eine Schnittstelle (serielle oder parallele Schnittstelle bei der EP 0 319 762 A1) oder eine Vernetzung (z.B. DE 92 18 373 U1) mit einem Steuerrechner verbunden, der das Gerät steuert und Betriebsparameter des Geräts auf einem Bildschirm anzeigt.

Weiterhin ist eine ähnliche Einrichtung, die allerdings zur Untersuchung und/oder Behandlung der Augen gedacht ist, aus der EP 0 568 081 A1 bekannt.

Die Verwendung serieller oder paralleler Schnittstellen zur Steuerung der Geräte den Nachteil, daß die Zahl der insgesamt durch den Steuerrechner steuerbaren Geräte durch die Zahl der Schnittstellen des Rechners begrenzt ist. Diese Zahl ist gerade bei einem Standard-PC vergleichsweise klein.

Die Vernetzung andererseits erfordert den Einsatz "vergleichsweise intelligenter" Geräte, so daß der Aufwand für die zentrale Steuerung auf der Geräteseite erhöht wird. Darüber hinaus ist es bei Standard-Vernetzungen, die beispielsweise auf der Basis von Netzwerk-Betriebssystemen, wie Novell erfolgen, vergleichsweise schwierig, Gerät während des Betriebs an- oder abzumelden. Dies kann bei medizinischen Eingriffen beispielsweise dann erforderlich sein, wenn ein Gerät ausfällt und durch ein anderes Gerät gleichen Typs ersetzt werden muß, oder eine bestimmte Operationssituation den Anschluß eines weiteren Gerätes erforderlich macht. Darüber hinaus sind die bekannten Netzwerklösungen nicht so ausfallsicher, daß sie im Operationssaal eingesetzt werden können.

Aber auch dann, wenn nur eine vergleichsweise kleine Zahl von Geräten zentral gesteuert wird, tritt immer dann, wenn mehr als ein Gerät gesteuert wird, folgendes Problem auf:

Die Bedienungsperson will die Betriebsparameter - beispielsweise - des Geräts 1 ändern, übersieht jedoch, daß sie sich im Steuerungsmode für - beispielsweise - das Gerät 2 befindet. Damit verstellt sie unbeabsichtigt die Betriebsparameter für dieses Gerät. Auch dann, wenn sie ihren Fehler sofort bemerkt, ist es häufig schwierig, die Betriebsparameter wieder auf "vernünftige" Werte "zurückzustellen".

### Darstellung der Erfindung

Der Erfindung liegt die Aufgabe zugrunde, eine Einrichtung zur zentralen Überwachung und/oder Steuerung wenigstens eines Gerätes für die Endoskopie und insbesondere für die minimal-invasive Chirurgie gemäß dem Oberbegriff des Patentanspruchs 1 derart weiterzubilden, daß bei vergleichsweise geringem Aufwand gerade bei den zu steuerenden Geräten eine große Zahl von (unterschiedlichen oder gleichen) Geräten zentral gesteuert werden kann, wobei der Ersatz ausgefallener Geräte oder das Anschließen neuer Geräte während des laufenden Betriebs problemlos und insbesondere ohne Störung der anderen Geräte möglich ist.

Weiterhin soll die erfindungsgemäße Einrichtung bevorzugt so ausgebildet sein, daß Bedienungsfehler einfach korrigiert und insbesondere rückgängig werden können, auch wenn bereits verschiedene andere Eingriffe in die Steuerung der Geräte vorgenommen worden sind.

Eine erfindungsgemäße Lösung dieser Aufgabe erhält man durch die im Anspruch 1 angegebene Kombination von Merkmalen. Weiterbildungen der Erfindung sind Gegenstand der Ansprüche 2 folgende.

Es ist dann auch möglich, Geräte während des Betriebs an- oder abzuschalten, so daß sich das "Netzwerk" beispielsweise wechselnden Operationssituationen anpassen kann. Insbesondere können auch ausgefallene Geräte problemlos ersetzt werden.

Insbesondere ist es von Vorteil, wenn der BUS sich automatisch während des Betriebs rekonfiguriert, wenn Geräte neu zugeschaltet oder abgeschaltet werden. Hierbei ist es weiterhin von Vorteil, wenn der Bus selbsttätig terminiert wird.

Als Bus können die verschiedensten Lösungen eingesetzt werden, eine Möglichkeit ist die Verwendung des sog. FireWire-Buses nach IEEE 1394. Eine weitere, bevorzugte Möglichkeit ist die Verwendung des CAN-Busses:

Der von der Fa. Robert Bosch, Deutschland, entwickelte und ISO sowie IEEE-genormte CAN-Bus (Controller Area Network) hat nicht nur die Vorteile einer hohen Störimmunität und einer einfachen Verdrahtung durch 2-Leiter-Technik, sondern vor allem den Vorteil, daß aufgrund des geringen Softwareaufwands auch Geräte mit einfachen 8-Bit Controllern angeschlossen werden können. Darüberhinaus ist der CAN-Bus echtzeitfähig und erlaubt eine Multimaster-Struktur. Zum Einsatz können sowohl die Versionen nach ISO 11898 als auch die Versionen nach ISO 11519/11898 kommen.

Aufgrund dieser Merkmale ist der Aufwand für die Modifizierung herkömmlicher Geräte zum Einsatz mit einer Einrichtung zur zentralen Steuerung vergleichsweise gering.

Dabei kann insbesondere sowohl die BUS-Arbitrierung als auch die Datenübertragung über eine Zweidrahtleitung erfolgen, so daß der Verdrahtungsaufwand besonders gering ist.

Anstelle einer "fest verdrahteten" Verbindung ist es in betimmten Fällen - beispielsweise beim Einsatz einer Fernsteuerung - bevorzugt, wenn die Busverbindung zumindest zwischen einem Teil der Geräte über eine Infrarot- oder Funk-Übertragungsstrecke erfolgt.

Die Stromversorgung der Bus-Schnittstelle erfolgt über die Busleitung, wobei die Buskommunikation nicht unterbrochen wird, wenn ein am Bus angeschlossenes Gerät ausgeschaltet wird, oder nicht eingeschaltet ist. Insbesondere kann dabei die Stromversorgung der Schnittstelle über die Busleitung mittels nicht zur Kommunikation benötigter Leitungen erfolgen. Weiterhin können ein oder mehrere Geräte die Schnittstellen der restlichen Geräte mit Strom versorgen.

Die Betriebssicherheit gerade beim Einsatz von HF-Geräten etc. wird weiter erhöht, wenn die Schnittstelle vom jeweiligen Gerät galvanisch getrennt ist.

Im Falle des Einsatzes einer Fernbedienung ist es von Vorteil, wenn die Fernbedienung mehr als ein Gerät steuern kann, und sich selbsttätig auf das jeweils zu steuernde Gerät konfiguriert. Dabei sollte die Fernbedienung derart aufgebaut sein, daß sie im Sterilbereich einsetzbar ist. Damit kann der behandelnde Arzt problemlos die Geräte selbst steuern, ohne auch eine Hilfsperon angewiesen zu sein. Um Fehlbedienungen zu vermeiden, ist es bevorzugt, wenn die von einer Bedingungsperson getätigten Änderungen und insbesondere die über die Fernsteuerung getätigten Änderungen graphisch oder gegebenenfalls akustisch angezeigt bzw. dargestellt werden.

Wie bereits ausgeführt, ist es von Vorteil, wenn der Bus multimaster-fähig ist. Dabei kann mindestens eines der folgenden Geräte als BUS-Master konfiguriert sein:
- Videosignal-Verarbeitungseinheit
- Steuerrechner
- Fernbedienungseinheit
- Netzwerk-Modul.
- Netzwerk-Modul.

Das Netzwerk-Modul kann an jedes an den Bus anschließbare Gerät anschließbar und insbesondere andockbar sein, so daß es nicht erforderlich ist, einen PC oder ein sonstiges Master-fähiges Steuergerät im Netzwerk einzusetzen. Die Verbindung des Netzwerk-Moduls kann insbesondere über eine serielle Schnittstelle erfolgen.

Wenn mehr als ein als BUS-Master verwendbares Gerät an den Bus angeschlossen ist, sollte bevorzugt durch eine Arbitrierung oder eine Prioritätszuweisung sichergestellt sein, daß nur ein BUS-Master aktiv die BUS-Master-Funktion übernimmt.

Bei einer bevorzugten Weiterbildung der Erfindung ist der BUS-Master ein Steuerrechner und insbesondere ein IBM-kompatibler bzw. ein sog. Industriestandard-PC mit einer Bus-Schnittstelle. Der Steuerrechner kann dabei insbesondere in einem Programmierungs- und in einem Anwendungsmodus arbeiten. Im Programmierungsmodus können die Betriebsparameter der zu steuernden Geräte voreingestellt und als Voreinstellung abgespeichert werden, während im Anwendungsmodus die Betriebsparameter nur aktuell ohne Beeinflussung der Grundeinstellung abgeändert werden können.

Hierdurch wird das Problem gelöst, daß bei einer zentralen Steuerung unbeabsichtigt das "falsche Gerät" bedient und damit verstellt wird.

Dabei ist es bevorzugt, wenn im Programmierungsmodus die Durchführung eines Behandlungsvorganges nicht möglich ist.

Damit hat die Bedienungsperson im Falle einer Fehlbedienung immer die Möglichkeit, zu der während der Operation, bei der sich die Einrichtung im Anwendungsmodus befindet, nicht veränderbaren Grundeinstellung "zurückzukehren". Diese Vorgehensweise ist wesentlich sicherer als die Ausgestaltung, bei der bei einer Verstellung eines Wertes der jeweils letzte Wert aufgerufen werden kann, da bereits dieser Wert durch eine Fehlbedienung ungewollt und für den jeweiligen Behandlungsvorgang unpassend verändert worden sein kann.

Im Hinblick auf die unterschiedlichen, beispielsweise bei Operationen auftretenden Fälle, ist es weiterhin bevorzugt, wenn für jedes zu steuernde Gerät mehrere unterschiedliche Voreinstellungen abgespeichert werden können, die von der Bedienungsperson entsprechend der jeweiligen Situation abgerufen werden können.

Dabei kann die Software so gestaltet sein, daß jedes an Bus angeschlossene Gerät individuell konfiguriert werden kann. Damit können beispielsweise verschiedene Geräte gleichen Typs individuell konfiguriert bzw. kalibriert werden.

Weiterhin ist es für die Protokollierung - gerade in Schadensfällen - von Vorteil, wenn die Software so gestaltet ist, das Benutzer der Einrichtung eindeutig identifizierbar ist. Dies kann beispielsweise dadurch erfolgen, daß der Benutzer beim Starten der erfindungsgemäßen Einrichtung seine Identifikation eingeben muß, und diese dann bei der Protokollierung mit abgespeichert wird.

Da verschiedene Benutzer häufig unterschiedliche "Vorlieben" für die Einstellung der einzelnen Geräte haben, kann bei einer weiteren vorteilhaften Ausgestaltung die Eingabe des jeweiligen Benutzers die Grundeinstellung der Einrichtung ändern.

Weiterhin kann die Software so gestaltet sein, daß die für einen bestimmten Vorgang benötigte Voreinstellung der am Bus angeschlossenen Geräte gespeichert werden kann.

Die Bedienung wird weiter vereinfacht, wenn der Steuerrechner eine graphische Benutzeroberfläche hat, die insbesondere intuitiv bedient werden kann. Derartige Benutzeroberflächen sind beispielsweise Windows 3.1, 95, NT oder Nachfolger oder die entsprechenden Oberflächen von OS/2 bzw. Unix-Systemen, wie z.B. Windows X. Selbstverständlich können auch andere Betriebssysteme wie OS9 etc. zum Einsatz kommen.

Die Bedienung der Geräte unter einer Streßsituation, wie sie gerade bei Operationen immer wieder auftritt, wird weiter erleichtert, wenn die auf einem Bildschirm des Steuerrechners dargestellte Benutzeroberfläche von der Bedienungsperson konfigurierbar ist. Damit können nur die gerade für den jeweiligen Operationsvorgang benötigten Bedienelemente dargestellt und die restlichen Bedienelemente ausgeblendet werden, so daß die Gefahr von Fehlbedienungen weiter verringert wird.

Um beispielsweise Benutzern, die bislang keine Erfahrung mit rechnergestützten Geräten haben, den Umstieg zu erleichtern, ist es weiterhin bevorzugt, wenn auf dem Bildschirm, auf dem die Benutzeroberfläche dargestellt ist, die Bedienelemente wenigstens eines herkömmlichen Geräts, wie Ein-/Aus-Schalter, Schiebe- oder Drehknöpfe sowie sonstige Funktionstasten graphisch nachgebildet sind. Dabei ist es insbesondere bevorzugt, wenn die dargestellten Bedienelemente in ihrem Aussehen den Bedienelementen herkömmlicher Geräte gleichen, da dann der Benutzer sich nicht umgewöhnen muß.

Weiterhin ist es möglich, daß auf dem Bildschirm die Bedienelemente mehrerer Geräte gleichzeitig dargestellt sind, so daß auch komplexe Behandlungsvorgänge einfach durchgeführt werden können.

Um die unbeabsichtigte Verstellung von Parametern zu vermeiden, ist es weiterhin von Vorteil, wenn lediglich eine von der Bedienungsperson konfigurierbare Auswahl von Bedienelementen des oder der Geräte gleichzeitig dargestellt ist. Dabei können insbesondere entsprechend der jeweils abgerufenen Voreinstellung lediglich bestimmte Parameter der an den Bus angeschlossenen Geräte verändert werden.

Die Bedienung des Steuerrechners kann beispielsweise mittels einer Maus, eines Joy-Sticks, einer Rollkugel, eines Touch-Screens oder dgl. erfolgen. In jedem Falle ist es von Vorteil, wenn die für die Bedienung vorgesehenen Elemente so ausgebildet sind, daß sie im Sterilbereich eingesetzt werden können. Dies kann beispielsweise dadurch erreicht werden, daß die Elemente selbst sterilisierbar sind, oder daß sie mit einem sterilen Überzug versehen werden.

Ferner ist es möglich, daß die über den Bus steuerbaren Funktionen durch ein Spracheingabe- und Verarbeitungsmodul sprachgesteuert werden. In diesem Falle ist es bevorzugt, wenn die Sprachkommandos auf einem Bildschirm graphisch angezeigt oder akustisch bestätigt werden, so daß die Bedienungsperson möglicherweise falsch verstandenen Kommandos erkennen und korrigieren kann. Dies wird dadurch erleichtert, wenn fehlerhaft eingegebene Kommandos bzw. Steuervorgänge unmittelbar rückgängig gemacht werden können.

Bei einer weiteren bevorzugten Weiterbildung der Erfindung ist eine Videosignal-Verarbeitungseinheit vorhanden, an der das Bildsignal einer digitalen oder analogen Videokamera angelegt ist, und die insbesondere eine Bildverarbeitungseinheit aufweisen kann. Die Videosignal-Verarbeitungseinheit kann dabei das von der Videokamera aufgenommene Bild in einem Fenster der graphischen Benutzeroberfläche des Steuerrechners darstellen.

Weiterhin ist es möglich, daß die Videossignal-Verarbeitungseinheit das von der Videokamera aufgenommene Bild digitalisiert und in einem Steuerrechner ablegt, in dem auch Patientenstammdaten sowie Geräteparameter in Zuordnung zu den gespeicherten Bildern gespeichert werden können. Dies ermöglicht nicht nur eine sichere Archivierung der gewonnenen Daten, sondern auch deren patientenspezifische Zuordnung und die Nachvollziehung von eventuellen Schadensfällen.

Der Steuerrechner kann über den Bus, also beispielsweise den CAN-Bus die Signalverarbeitung in der Videoeinheit steuern. Hierzu kann die Videoeinheit einen Mikroprozessor aufweisen, der die Videoeignalverarbeitung sowie gegebenenfalls eine Graphikprozessor steuert, der Daten und/oder ein Overlay-Bild in das aktuell aufgenommene Videobild einblendet.

Insbesondere können die Videokamera und der Videomonitor direkt und nicht über den CAN-Bus an der Videoeinheit angeschlossen sein.

Die Videosignal-Verarbeitungseinheit kann das von der Videokamera aufgenommene Bild auf einem Analog-Monitor und gegebenenfalls zusätzlich die über den Bus empfangenen Informationen in einem Video-Overlay auf dem Analog-Monitor darstellen. Weiterhin ist die Darstellung auf einem Monitor des Steuerrechners in einem Vidoe-Overlay oder in einem Fenster möglich, in dem ein digitalisiertes Bild dargestellt wird. Das Video-Overlay kann dabei vom Anwender konfigurierbar sein.

Darüber hinaus können die über die Fernsteuerung getätigten Einstellungsänderung auf dem Bildschirm des Steuerrechners oder mittels eines Video-Overlays graphisch angezeigt werden.

Bei einer bevorzugten Weiterbildung der Erfindung sind mindestens zwei Geräte über den Bus zu einem geschlossenen Regelkreis verbunden, der insbesondere Geräte-interne Regelungen überlagern kann. Zur Vermeidung von "Interferenzen" ist es bevorzugt, wenn der über den Bus vermittelte Regelkreis eine wesentlich langsamere Zeitkonstante als die Geräte-interne Regelung hat.

Die über den Bus zu einem geschlossenen Regelkreis verbundenen Geräte können insbesondere
- eine Insufflations- und eine Absaug-Einrichtung
- eine Lichtquelle und eine Videokamera, und /oder
- eine Druckmeßeinheit und eine Pumpe bzw. ein Insufflator
sein.

In jedem Falle ist es bevorzugt, wenn die Software so gestaltet ist, daß ein Ausfall der Software oder der Hardware dem Anwender sicher angezeigt wird, so daß ein Standard-Betriebssystem und Standard Hardware ohne Sicherheitseinbussen eingesetzt werden können.

### Kurze Beschreibung der Zeichnung

Die Erfindung wird nachstehend exemplarisch anhand der Figuren der Zeichnung näher beschrieben, in der zeigen:
- Fig. 1a-d: verschiedene Netzwerk-Konfigurationen,
- Fig. 2: eine erfindungsgemäße Einrichtung mit Video-Signalverarbeitung, und
- Fig. 3: eine Realisierung des erfindungsgemäßen Sicherheitskonzepts.

### Darstellung von Ausführungsbeispielen

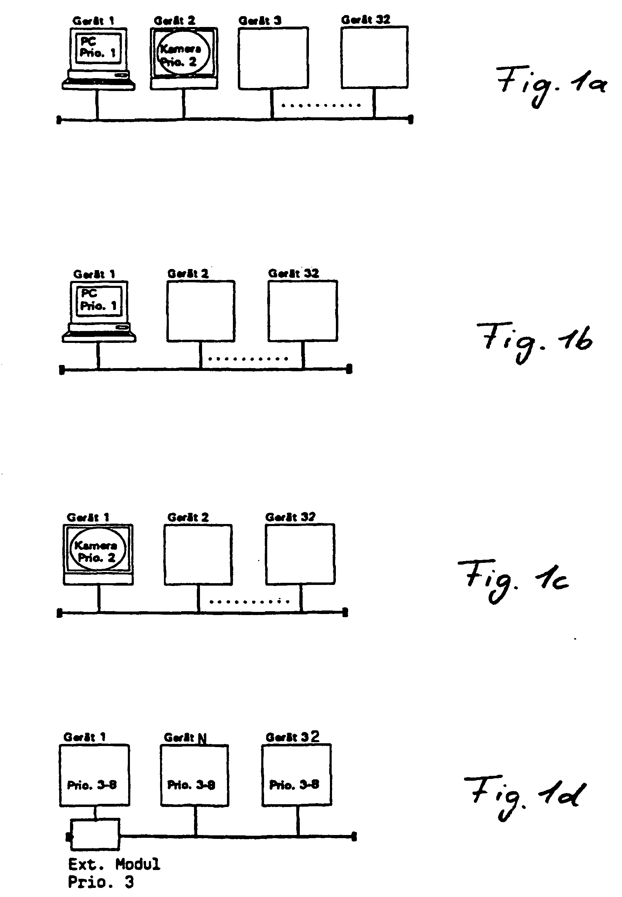

In Fig. 1 sind verschiedene Möglichkeiten für die Konfiguration des erfindungsgemäß verwendeten Netzwerkes dargestellt.

Fig. la zeigt als erste Möglichkeit den Fall, daß ein Steuerrechner PC (Gerät 1) als Netzwerk-Master dient, während eine Kamerasteuereinheit bzw. eine Videosignalverarbeitungseinheit (Gerät 2) als Anzeigegerät dient. Die Geräte 3 bis 32, beispielsweise Insufflatoren, Pumpen, HF-Chirurgiegeräte, Lichtquellen etc. sind als Netzwerk-Slaves geschaltet.

Fig. 1b zeigt als zweite Möglichkeit den Fall, daß ein Steuerrechner PC (Gerät 1) als Netzwerk-Master und als Anzeigegerät dient, während alle anderen Geräte 2 bis 32 als Netzwerk-Slaves geschaltet sind.

Fig. 1c zeigt als dritte Möglichkeit den Fall, daß die Kamerasteuereinheit (Gerät 1) sowohl als Netzwerk-Master als auch als Anzeigegerät dient, wären alle anderen Geräte 2 bis 32 wiederum des Netzwerk-Slaves geschaltet sind.

Fig. 1d zeigt den Fall, daß ein externes Schnittstellenmodul als Netzwerk-Master geschaltet ist, ohne daß ein Anzeigegerät vorhanden wäre. Die Geräte 1 bis 32 sind als Netzwerk-Slaves geschaltet.

Für den Fall, daß der Steuerrechner (PC), die Kamerasteuereinheit (Kamera) und das externe Schnittstellenmodul gleichzeitig in einem Netzwerk vorhanden sind, sind diese Elemente mit unterschiedlichen Prioritäten versehen, so daß immer nur ein Gerät als Bus-Master fungiert. Bei dem gezeigten Ausführungsbeispiel hat der Steuerrechner Priorität vor der Kamerasteuereinheit und dem Schnittstellenmodul und die Kamerasteuereinheit Vorrang als Bus-Master vor dem Schnittstellenmodul.

Fig. 2 zeigt ein Ausführungsbeispiel einer erfindungsgemäßen Einrichtung mit Videosignalverarbeitung. Ein Mikroprozessor bzw. ein Mikrocontroller µP dient als zentrale Steuereinheit. Über eine RS 232 Schnittstelle - gegebenenfalls auch ein FIFO oder ein Dual Ported RAM - ist der Mikroprozessor µP mit einem Schnittstellenmodul (SCB-Modul) verbunden, das entsprechend dem gewählten Bus, also beispielsweise einem CAN-Bus ausgebildet ist, und das über Elemente zur Bus-Steuerung und - Kommunikation sowie gegebenenfalls über Elemente zur galvanischen Trennung, wie Optokoppler verfügt. Zur Steuerung des Mikroprozessors µP ist bei dem gezeigten Ausführungsbeispiel eine Tastatur (keyboard) vorgesehen.

Der Mikroprozessor µP steuert einerseits einen Videosignalprozessor (Videosignal Processing), an dem das Kamerasignal anliegt, und empfängt andererseits von dem Videosignalprozessor Daten, die er beispielsweise speichert und/oder über den Bus in einer Patientendatei auf einem Rechner ablegt.

Ferner steuert der Videosignalprozessor einen Graphikprozessor (Graphics Processor, Title Generator), der u.a. ein Video-Overlay, das vom Anwender konfigurierbar ist, auf einem nicht dargestellten Monitor erzeugen kann.

Die Ausgangssignale des Videosignalprozessors und des Graphikprozessors werden überlagert als Video-Ausgangssignal unabhängig von dem Bus ausgegeben und beispielsweise auf einem Analog- oder Digitalmonitor dargestellt und/oder auf einem Rechner abgespeichert.

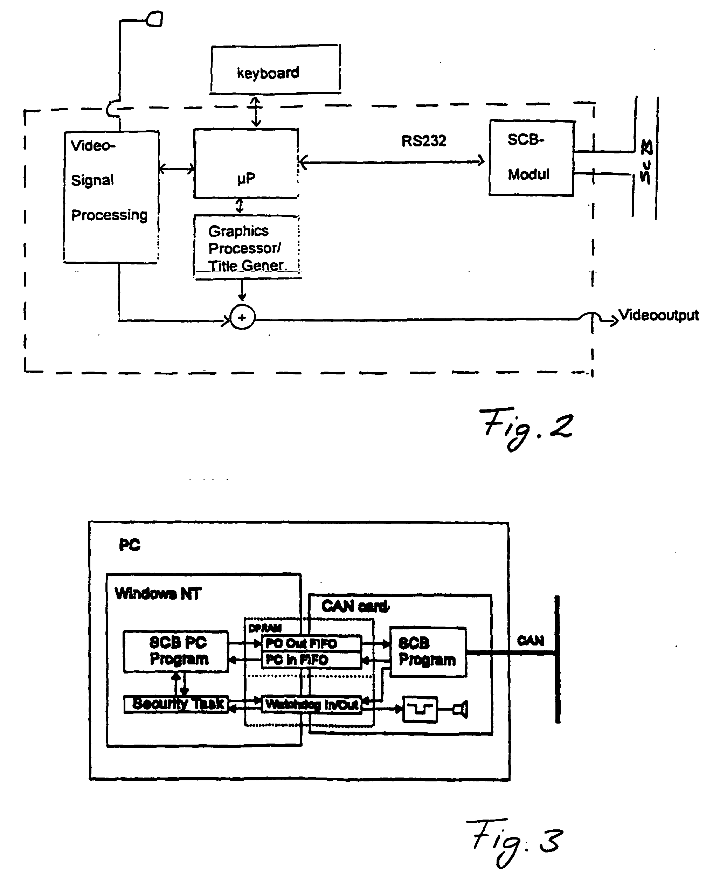

Fig. 3 zeigt eine Realisierung des erfindungsgemäßen Sicherheitskonzepts. Ein Steuerrechner (PC), der beispielsweise unter Windows NT das Bus-Master-Steuerprogramm ausführt, ist über ein FIFO oder ein Dual-Ported RAM mit einer Schnittstellenkarte (CAN card) verbunden. Zusätzlich ist ein sog. Security-Task vorgesehen der zyklisch Nachrichten zwischen dem Steuerrechner und der Schnittstellenkarte austauscht. Wird in dem Security-Task festgestellt, daß die Datenkommunikation gestört ist, wird ein Alarm ausgegeben. Hierzu ist die Schnittstellenkarte mit einem äkustischen Alarmgeber versehen. Im übrigen wird zum Aufbau auf Fig. 3 verwiesen.

Vorstehend ist die Erfindung anhand von Ausführungsbeispielen ohne Beschränkung des allgemeinen Erfindungsgedankens beschrieben worden. Innerhalb dieses - den Ansprüchen entnehmbaren - Erfindungsgedankens sind selbstverständlich die verschiedensten Abwandlungen möglich:

So können die verschiedensten Bus-Systeme eingesetzt werden, solange diese selbstkonfigurierend sind, und der BUS-Master den Bus automatisch konfiguriert und überwachen kann, ob die Kommunikation auf dem Bus korrekt abläuft.

## Patentansprüche

1. Einrichtung zur zentralen Überwachung und Steuerung wenigstens eines Gerätes für die Endoskopie, bei der das oder die zu steuernden Geräte über Bus-Schnittstellen miteinander verbunden sind, wobei in der Einrichtung
- die Geräte über die Bus-Schnittstellen an einen selbstkonfigurierenden Bus mit einem Bus-Master angeschlossen sind,
- der Bus-Master den Bus automatisch konfiguriert,
- der Bus-Master überwacht, ob die Kommunikation auf dem Bus korrekt abläuft,
**dadurch gekennzeichnet,**
**dass** die Stromversorgung der Bus-Schnittstelle über die Busleitung erfolgt, wobei die Buskommunikation nicht unterbrochen wird, wenn ein am Bus angeschlossenes Gerät ausgeschaltet wird oder nicht eingeschaltet ist.

2. Einrichtung nach Anspruch 1, die für die minimal-invasive Chirurgie und zur Überwachung oder Steuerung einer Insufflationsvorrichtung, einer Pumpe, einer Lichtquelle und/oder einer Videokamera ausgebildet ist.

3. Einrichtung nach Anspruch 1 oder 2,
**dadurch gekennzeichnet, daß** der selbstkonfigurierende Bus ein CAN-Bus ist.

4. Einrichtung nach einem der Ansprüche 1 bis 3,
**dadurch gekennzeichnet, daß** die BUS-Arbitrierung sowie die Datenübertragung über eine Zweidrahtleitung erfolgt.

5. Einrichtung nach einem der Ansprüche 1 bis 4,
**dadurch gekennzeichnet, daß** die Busverbindung zumindest zwischen einem Teil der Geräte über eine Infrarot- oder Funk-Übertragungsstrecke erfolgt.

6. Einrichtung nach einem der Ansprüche 1 bis 5,
**dadurch gekennzeichnet, daß** der Bus selbsttätig terminiert wird.

7. Einrichtung nach einem der Ansprüche 1 bis 6,
**dadurch gekennzeichnet, daß** die Stromversorgung der Schnittstelle über die Busleitung mittels nicht zur Kommunikation benötigter Leitungen erfolgt.

8. Einrichtung nach einem der Ansprüche 1 bis 7,
**dadurch gekennzeichnet, daß** die Schnittstelle vom jeweiligen Gerät galvanisch getrennt ist.

9. Einrichtung nach einem der Ansprüche 1 bis 8,
**dadurch gekennzeichnet, daß** eines der an den Bus angeschlossenen Geräte eine Fernbedienung für wenigstens ein weiteres Gerät ist.

10. Einrichtung nach Anspruch 9,
**dadurch gekennzeichnet, daß** die Fernbedienung mehr als ein Gerät steuern kann, und sich selbsttätig auf das jeweils zu steuernde Gerät konfiguriert.

11. Einrichtung nach Anspruch 9 oder 10,
**dadurch gekennzeichnet, daß** die Fernbedienung derart aufgebaut ist, daß sie im Sterilbereich einsetzbar ist.

12. Einrichtung nach einem der Ansprüche 9 bis 11,
**Dadurch gekennzeichnet, daß** die über die Fernsteuerung getätigten Änderungen der Einstellung von Geräten graphisch angezeigt werden.

13. Einrichtung nach einem der Ansprüche 1 bis 12,
**dadurch gekennzeichnet, daß** mindestens eines der folgenden Geräte als BUS-Master konfiguriert ist:
- Videosignal-Verarbeitungseinheit
- Steuerrechner
- Fernbedienungseinheit
- Netzwerk-Modul.

14. Einrichtung nach Anspruch 13,
**dadurch gekennzeichnet, daß** das Netzwerk-Modul an jedes an den Bus anschließbare Gerät anschließbar und andockbar ist.

15. Einrichtung nach einem der Ansprüche 1 bis 14,
**dadurch gekennzeichnet, daß** dann, wenn mehr als ein als BUS-Master verwendbares Gerät an den Bus angeschlossen ist, durch eine Arbitrierung sichergestellt ist, daß nur ein BUS-Master aktiv die BUS-Master-Funktion übernimmt.

16. Einrichtung nach einem der Ansprüche 1 bis 15,
**dadurch gekennzeichnet, daß** der BUS-Master ein Steuerrechner und ein Industriestandard-PC mit einer Bus-Schnittstelle ist.

17. Einrichtung nach Anspruch 16,
**dadurch gekennzeichnet, daß** der Steuerrechner in einem Programmierungs- und in einem Anwendungs-bzw. Kommunikationsmodus arbeitet.

18. Einrichtung nach Anspruch 16,
**dadurch gekennzeichnet, daß** im Programmierungsmodus die Betriebsparameter der zu steuernden Geräte voreingestellt und als Voreinstellung abgespeichert werden können, und
daß im Anwendungsmodus die Betriebsparameter nur aktuell ohne Beeinflussung der Grundeinstellung abgeändert werden können.

19. Einrichtung nach Anspruch 18,
**dadurch gekennzeichnet, daß** für jedes zu steuernde Gerät mehrere unterschiedliche Voreinstellungen abgespeichert werden können, die von der Bedienungsperson entsprechend der jeweiligen Situation abgerufen werden können.

20. Einrichtung nach Anspruch 18 oder 19,
**dadurch gekennzeichnet, daß** im Programmierungsmodus die Durchführung eines Behandlungsvorganges nicht möglich ist.

21. Einrichtung nach einem der Ansprüche 16 bis 20,
**dadurch gekennzeichnet, daß** der Steuerrechner eine graphische Benutzeroberfläche hat.

22. Einrichtung nach Anspruch 21,
**dadurch gekennzeichnet, daß** die auf einem Bildschirm des Steuerrechners dargestellte Benutzeroberfläche von der Bedienungsperson konfigurierbar ist.

23. Einrichtung nach Anspruch 20 oder 21,
**dadurch gekennzeichnet, daß** auf dem Bildschirm, auf dem die Benutzeroberfläche dargestellt ist, die Bedienelemente wenigstens eines herkömmlichen Geräts, wie Ein-/Aus-Schalter, Schiebe- oder Drehknöpfe sowie sonstige Funktionstasten graphisch nachgebildet sind.

24. Einrichtung nach Anspruch 23,
**dadurch gekennzeichnet, daß** auf dem Bildschirm die Bedienelemente mehrerer Geräte gleichzeitig dargestellt sind.

25. Einrichtung nach Anspruch 23 oder 24,
**dadurch gekennzeichnet, daß** lediglich eine von der Bedienungsperson konfigurierbare Auswahl von Bedienelementen des oder der Geräte gleichzeitig dargestellt ist.

26. Einrichtung nach Anspruch 29,
**dadurch gekennzeichnet, daß** entsprechend der jeweils abgerufenen Voreinstellung lediglich bestimmte Parameter der an den Bus angeschlossenen Geräte verändert werden können.

27. Einrichtung nach einem der Ansprüche 16 bis 26,
**dadurch gekennzeichnet, daß** die Bedienung des Steuerrechners mittels einer Maus, eines Joy-Sticks, einer Rollkugel erfolgt.

28. Einrichtung nach einem der Ansprüche 16 bis 26,
**dadurch gekennzeichnet, daß** die Bedienung des Steuerrechners mittels eines Touch-Screens erfolgt.

29. Einrichtung nach Anspruch 27 oder 28,
**dadurch gekennzeichnet, daß** die Bedienelemente derart ausgebildet sind, daß eine Bedienung im Sterilbereich möglich ist

30. Einrichtung nach einem der Ansprüche 1 bis 29,
**dadurch gekennzeichnet, daß** die über den Bus steuerbaren Funktionen durch ein Spracheingabe- und Verarbeitungsmodul sprachgesteuert werden.

31. Einrichtung nach Anspruch 30,
**dadurch gekennzeichnet, daß** die Sprachkommandos auf einem Bildschirm graphisch angezeigt oder akustisch bestätigt werden.

32. Einrichtung nach Anspruch 31,
**dadurch gekennzeichnet, daß** fehlerhaft eingegebene Kommandos bzw. Steuervorgänge unmittelbar rückgängig gemacht werden können.

33. Einrichtung nach einem der Ansprüche 1 bis 32
**dadurch gekennzeichnet, daß** eine Videosignal-Verarbeitungseinheit vorhanden ist, an der das Bildsignal einer Videokamera angelegt ist.

34. Einrichtung nach Anspruch 33,
**dadurch gekennzeichnet, daß** die Videokamera eine analoge oder digitale Videokamera ist.

35. Einrichtung nach Anspruch 33 oder 34,
**dadurch gekennzeichnet, daß** die Videosignal-Verarbeitungseinheit eine Bildverarbeitungseinheit aufweist.

36. Einrichtung nach einem der Ansprüche 33 bis 35,
**dadurch gekennzeichnet, daß** die Videosignal-Verarbeitungseinheit das von der Videokamera aufgenommene Bild digitalisiert und in einem Fenster der graphischen Benutzeroberfläche des Steuerrechners darstellt.

37. Einrichtung nach einem der Ansprüche 33 bis 36,
**dadurch gekennzeichnet, daß** die Videossignal-Verarbeitungseinheit das von der Videokamera aufgenommene Bild digitalisiert und in einem Steuerrechner ablegt, in dem auch Patientenstammdaten sowie Geräteparameter in Zuordnung zu den gespeicherten Bildern gespeichert werden können.

38. Einrichtung nach einem der Ansprüche 33 bis 37,
**dadurch gekennzeichnet, daß** die Videosignal-Verarbeitungseinheit das von der Videokamera aufgenommene Bild auf einem Analog-Monitor oder dem Monitor des Steuerrechners darstellt.

39. Einrichtung nach einem der Ansprüche 33 bis 38,
**dadurch gekennzeichnet, daß** die Videosignal-Verarbeitungseinheit die über den Bus empfangenen Informationen in einem Video-Overlay auf dem Analog-Monitor darstellt.

40. Einrichtung nach Anspruch 39,
**dadurch gekennzeichnet, daß** das Video-Overlay vom Anwender konfigurierbar ist.

41. Einrichtungen nach Anspruch 40,
**dadurch gekennzeichnet, daß** das Video-Overlay Geräte- und/oder Behandlungsparameter und/oder Fehlermeldungen anzeigt.

42. Einrichtung nach einem der Ansprüche 33 bis 41,
**dadurch gekennzeichnet, daß** die Videosignal-Verarbeitungseinheit einen Mikroprozessor aufweist, der die Videosignalverarbeitung sowie gegebenenfalls eine Graphikprozessor steuert, der Daten und/oder das Overlay-Bild in das aktuell aufgenommene Videobild einblendet.

43. Einrichtung nach einem der Ansprüche 1 bis 42,
**dadurch gekennzeichnet, daß** die über die Fernsteuerung getätigten Einstellungsänderung auf dem Bildschirm des Steuerrechners oder mittels eines Video-Overlays graphisch angezeigt werden.

44. Einrichtung nach einem der Ansprüche 1 bis 43,
**dadurch gekennzeichnet, daß** die Videokamera und der Videomonitor direkt und nicht über den Bus an der Videosignal-Verarbeitungseinheit angeschlossen sind.

45. Einrichtung nach einem der Ansprüche 1 bis 44,
**dadurch gekennzeichnet, daß** mindestens zwei Geräte über den Bus zu einem geschlossenen Regelkreis verbunden sind.

46. Einrichtung nach Anspruch 45,
**dadurch gekennzeichnet, daß** der über den Bus vermittelte Regelkreis Geräte-interne Regelungen überlagert.

47. Einrichtung nach Anspruch 46,
**dadurch gekennzeichnet, daß** der über den Bus vermittelte Regelkreis eine wesentlich langsamere Zeitkonstante als die Geräte-interne Regelung hat.

48. Einrichtung nach einem der Ansprüche 45 bis 47,
**dadurch gekennzeichnet, daß** die über den Bus zu einem geschlossenen Regelkreis verbundenen Geräte
- eine Insufflations- und eine Absaug-Einrichtung
- eine Lichtquelle und eine Videokamera, und /oder
- eine Druckmeßeinheit und eine Pumpe bzw. ein Insufflator sind.

49. Einrichtung nach einem der Ansprüche 1 bis 48,
**dadurch gekennzeichnet, daß** die Bus-Karte über eine serielle Schnittstelle, ein FIFO oder ein Dual Ported RAM mit dem Steuerrechner Daten austauscht.

50. Einrichtung nach Anspruch 49,
**dadurch gekennzeichnet, daß** eine Überwachungsfunktion vorgesehen ist, die bei einem Ausfall des Steuerrechners oder der Bus-Karte einen Alarm auslöst.

51. Einrichtung nach einem der Ansprüche 1 bis 50,
**dadurch gekennzeichnet, daß** die Software so gestaltet ist, daß ein Ausfall der Software oder der Hardware dem Anwender sicher angezeigt wird, so daß ein Standard-Betriebssystem und Standard Hardware ohne Sicherheitseinbussen eingesetzt werden können.

52. Einrichtung nach einem der Ansprüche 1 bis 51,
**dadurch gekennzeichnet, daß** die Software so gestaltet ist, das Benutzer der Einrichtung eindeutig identifizierbar ist.

53. Einrichtung nach Anspruch 52,
**dadurch gekennzeichnet, daß** die Eingabe des jeweiligen Benutzers die Grundeinstellung der Einrichtung ändert.

54. Einrichtung nach einem der Ansprüche 1 bis 53,
**dadurch gekennzeichnet, daß** die Software so gestaltet ist, daß jedes an Bus angeschlossene Gerät individuell konfiguriert werden kann.

55. Einrichtung nach einem der Ansprüche 1 bis 54,
**dadurch gekennzeichnet, daß** die Software so gestaltet ist, daß die für einen bestimmten Vorgang benötigte Voreinstellung der am Bus angeschlossenen Geräte gespeichert werden kann.

## Claims

1. A device for the central monitoring and control of at least one endoscopic appliance, in which the appliance(s) to be controlled are connected to one another via bus interfaces, and where in the device
- the appliances are connected via the bus interfaces to a self-configuring bus with a bus master,
- the bus master automatically configures the bus,
- the bus master monitors whether the communication on the bus is running correctly, -
**characterised in that** power is supplied to the bus interface via the bus line, whereby the bus communication is not interrupted when an appliance connected to the bus is switched off or is not switched on.

2. Device according to Claim 1, designed for minimally invasive surgery and for monitoring or controlling an insufflation device, a pump, a light source and/or a video camera.

3. Device according to Claim 1 or 2,
**characterised in that** the seif-configuring bus is a CAN bus.

4. Device according to any of Claims 1 to 3,
**characterised in that** bus arbitration and data transfer take place via a two-wire connection.

5. Device according to any of Claims 1 to 4,
**characterised in that** the bus connection takes place at least between part of the appliances via an infra-red or radio transmission link.

6. Device according to any of Claims 1 to 5,
**characterised in that** the bus is terminated automatically.

7. Device according to any of Claims 1 to 6,
**characterised in that** power is supplied to the interface via the bus line using supply lines not required for communication.

8. Device according to any of Claims 1 to 7,
**characterised in that** the interface is galvanically separated from the appliance in question.

9. Device according to any of Claims 1 to 8,
**characterised in that** one of the appliances connected to the bus is a remote control for at least one further appliance.

10. Device according to Claim 9,
**characterised in that** the remote control can control more than one appliance, and automatically configures itself to the appliance which is to be controlled.

11. Device according to Claim 9 or 10,
**characterised in that** the remote control is constructed in such a way that it can be used in the sterile area.

12. Device according to any of Claims 9 to 11,
**characterised in that** the alterations to the appliances' settings made via the remote control are graphically displayed.

13. Device according to any of Claims 1 to 12,
**characterised in that** at least one of the following appliances is configured as a bus master:
- video signal processing unit
- control computer
- remote control unit
- network module.

14. Device according to any of Claims 13,
**characterised in that** the network module of each appliance which can be connected to the bus can be connected and docked.

15. Device according to any of Claims 1 to 14,
**characterised in that**, when more than one of the appliances which can be used as a bus master is connected to the bus, arbitration ensures that only one bus master actively assumes the function of a bus master.

16. Device according to any of Claims 1 to 15,
**characterised in that** the bus master is a control computer and an industry-standard PC with a bus interface.

17. Device according to Claim 16,
**characterised in that** the control computer works in a program mode and an application or communication mode.

18. Device according to Claim 16,
**characterised in that**, when in programming mode, the operating parameters of the appliances to be controlled can be pre-set and are stored as defaults, and that, when in application mode, the operating parameters can only be altered at the time, without affecting the basic settings.

19. Device according to Claim 18,
**characterised in that** several different pre-sets can be saved for each appliance to be controlled, and that these settings can be retrieved by the operator according to the situation which arises.

20. Device according to Claim 18 or 19,
**characterised in that** in program mode no treatment procedure can be carried out.

21. Device according to any of Claims 16 to 20,
**characterised in that** the control computer has a graphical user interface.

22. Device according to Claim 21,
**characterised in that** the user interface displayed on the control computer's screen can be configured by the user.

23. Device according to Claim 20 or 21,
**characterised in that** at least the user elements of a conventional appliance, such as on and off switches, sliding switches and knobs and other function keys, are graphically displayed on the user interface screen.

24. Device according to Claim 23,
**characterised in that** the user elements of more than one appliance can be displayed on the screen simultaneously.

25. Device according to Claim 23 or 24,
**characterised in that** only one of the sets of appliance user functions which can be configured by the operator is displayed at any one time.

26. Device according to Claim 25,
**characterised in that** only certain parameters of the applications connected to the bus, depending on the preset value selected, can be altered.

27. Device according to any of Claims 16 to 26,
**characterised in that** the control computer is operated using a mouse, a joystick, a track ball or a touch screen.

28. Device according to any of Claims 16 to 27,
**characterised in that** the control computer is operated using a touch screen.

29. Device according to Claim 27 or 28,
**characterised in that** the control elements are constructed in such a way that they can be used in the sterile area.

30. Device according to any of Claims 1 to 29,
**characterised in that** the functions controlled via the bus are controlled by means of a voice data entry and processing module.

31. Device according to Claim 30,
**characterised in that** the speech commands are graphically displayed on a screen or confirmed acoustically.

32. Device according to Claim 31,
**characterised in that** incorrectly entered commands or control procedures can be cancelled immediately.

33. Device according to any of Claims 1 to 32,
**characterised in that** there is a video signal processing unit to which the picture signal of a video camera is applied.

34. Device according to Claim 33,
**characterised in that** the video camera is an analog or digital video camera.

35. Device according to Claim 33 or 34,
**characterised in that** the video signal processing unit includes an image processing unit.

36. Device according to any of Claims 33 to 35,
**characterised in that** the video signal processing unit digitalizes the image received from the video camera and displays it in a window on the control computer's user interface.

37. Device according to any of Claims 33 to 36,
**characterised in that** the video signal processing unit digitalizes the image from the video camera and stores it in a control computer, where patient data and appliance parameters can be saved and matched to the stored images.

38. Device according to any of Claims 33 to 37,
**characterised in that** the video signal processing unit displays the image recorded by the video camera on an analog monitor or the control computer's monitor.

39. Device according to any of Claims 33 to 38,
**characterised in that** the video signal processing unit displays the information received via the bus in a video overlay on the analog monitor.

40. Device according to Claim 39,
**characterised in that** the video overlay can be configured by the user.

41. Device according to Claim 40,
**characterised in that** the video overlay displays appliance and/or treatment parameters and/or error messages.

42. Device according to any of Claims 33 to 41,
**characterised in that** the video signal processing unit has a microprocessor micro processor which controls the video signal processing and, if required, a graphics processor, which can superimpose the data or an overlay image on the current video image.

43. Device according to any of Claims 1 to 42,
**characterised in that** alterations to the settings made with the remote control are graphically displayed on the control computer's screen or by means of a video overlay.

44. Device according to any of Claims 1 to 43,
**characterised in that** the video camera and the video monitor are connected directly to the video signal processing unit, rather than via the CAN bus.

45. Device according to any of Claims 1 to 44,
**characterised in that** at least two appliances are connected via the bus to form a closed control loop.

46. Device according to Claim 45,
**characterised in that** the control loop relayed via the bus can be superimposed on the device-internal control systems.

47. Device according to Claim 46,
**characterised in that** the control loop relayed via the bus has a considerably slower time constant than the appliance-internal control system.

48. Device according to any of Claims 45 to 47,
**characterised in that** the appliances connected via the bus to form a closed control loop are
- an insufflation device and a suction device
- a light source and a video camera, and/or
- a pressure gauge and a pump or an insufflator.

49. Device according to any of Claims 1 to 48,
**characterised in that** the bus card exchanges data with the control computer via a serial interface, a FIFO or dual-ported RAM.

50. Device according to Claim 49,
**characterised in that** here is a monitoring function which sets off an alarm if the control computer or bus card fails.

51. Device according to any of Claims 1 to 50,
**characterised in that** the software is designed in such a way that the user is securely notified if the software or hardware fails, meaning that a standard operating system and standard hardware can be used without safety being compromised in any way.

52. Device according to any of Claims 1 to 51,
**characterised in that** the software is designed in such a way that the user of the device can be clearly identified.

53. Device according to Claim 52,
**characterised in that** the basic settings of the appliance are changed when each user is identified.

54. Device according to any of Claims 1 to 53,
**characterised in that** the software is designed in such a way that every appliance connected to the bus can be individually configured.

55. Device according to any of Claims 1 to 54,
**characterised in that** the software is designed in such a way that the default settings of the appliances connected to the bus required for a particular procedure can be stored.

## Revendications

1. Installation de surveillance et de commande centrale d'au moins un appareil pour l'endoscopie, dans laquelle le ou les appareils à commander sont reliés entre eux via des interfaces bus, moyennant quoi dans l'installation,
- les appareils sont raccordés à un bus autoconfigurant avec un bus master via les interfaces bus,
- le bus master configure automatiquement le bus,
- le bus master surveille si la communication se déroule correctement sur le bus,
**caractérisée en ce que** l'alimentation en courant de l'interface bus s'effectue via la ligne du bus, la communication du bus n'étant pas interrompue lorsqu'un appareil raccordé au bus est déconnecté ou n'est pas connecté.

2. Installation selon la revendication 1, qui est réalisée pour la chirurgie à invasion minimale et en vue de la surveillance ou de la commande d'un dispositif d'insufflation, d'une pompe, d'une source de lumière et/ou d'une caméra vidéo.

3. Installation selon la revendication 1 ou 2, **caractérisée en ce que** le bus autoconfigurant est un bus CAN.

4. Installation selon l'une quelconque des revendications 1 à 3, **caractérisée en ce que** l'arbitrage du bus ainsi que le transfert de données s'effectue via une ligne bifilaire.

5. Installation selon l'une quelconque des revendications 1 à 4, **caractérisée en ce que** le raccordement bus s'effectue pour le moins entre une partie des appareils via un trajet de transmission infrarouge ou radioélectrique.

6. Installation selon l'une quelconque des revendications 1 à 5, **caractérisée en ce que** le bus est auto actualisé.

7. Installation selon l'une quelconque des revendications 1 à 6, **caractérisée en ce que** l'alimentation en courant de l'interface s'effectue via la ligne du bus au moyen de lignes non nécessaires à la communication.

8. Installation selon l'une quelconque des revendications 1 à 7, **caractérisée en ce que** l'interface est galvaniquement séparée de l'appareil concerné.

9. Installation selon l'une quelconque des revendications 1 à 8, **caractérisée en ce qu'**un des appareils raccordés au bus est une commande à distance pour au moins un autre appareil.

10. Installation selon la revendication 9, **caractérisée en ce que** la commande à distance peut contrôler plus d'un appareil, et se configure automatiquement sur chacun des appareils à contrôler.

11. Installation selon la revendication 9 ou 10, **caractérisée en ce que** la commande à distance est constituée de telle manière qu'elle est utilisable en zone stérile.

12. Installation selon l'une quelconque des revendications 9 à 11, **caractérisée en ce que** les modifications de réglage d'appareils réalisées via la commande à distance sont affichées graphiquement.

13. Installation selon l'une quelconque des revendications 1 à 12, **caractérisée en ce qu'**au moins un des appareils suivants est configuré sous forme de bus master :
- Unité de traitement de signaux vidéo
- Calculateur de commande
- Unité de commande à distance
- Module de réseau.

14. Installation selon la revendication 13, **caractérisée en ce que** le module de réseau peut être raccordé et amarré à chaque appareil raccordable au bus.

15. Installation selon l'une quelconque des revendications 1 à 14, **caractérisée en ce que** dans le cas où plus d'un appareil utilisable en tant que bus master est raccordé au bus, il est garanti grâce à un arbitrage que seul un bus master assume activement la fonction de bus master.

16. Installation selon l'une quelconque des revendications 1 à 15, **caractérisée en ce que** le bus master est un calculateur de commande et un ordinateur personnel du standard de l'industrie avec une interface de bus.

17. Installation selon la revendication 16, **caractérisée en ce que** le calculateur de commande fonctionne dans un mode de programmation et dans un mode d'application ou de communication.

18. Installation selon la revendication 16, **caractérisée en ce que** dans le mode de programmation, les paramètres de fonctionnement des appareils à commander peuvent être préréglés et être mis en mémoire sous forme de réglage par défaut, et **en ce que** dans le mode d'application, les paramètres de fonctionnement peuvent être modifiés uniquement ponctuelle sans influencer le réglage de base.

19. Installation selon la revendication 18, **caractérisée en ce que** pour chaque appareil à commander, plusieurs réglages par défaut différents peuvent être mis en mémoire et peuvent être appelés par l'opérateur en fonction de la situation respective.

20. Installation selon la revendication 18 ou 19, **caractérisée en ce que** dans le mode de programmation, la réalisation d'un processus de traitement n'est pas possible.

21. Installation selon l'une quelconque des revendications 16 à 20, **caractérisée en ce que** l'ordinateur de commande dispose d'une interface utilisateur graphique.

22. Installation selon la revendication 21, **caractérisée en ce que** l'interface utilisateur visualisée sur un écran du calculateur de commande est configurable par l'opérateur.

23. Installation selon la revendication 20 ou 21, **caractérisée en ce que** les éléments de réglage d'au moins un appareil traditionnel, tel que des contacteurs/coupe-circuits, des boutons tournants ou coulissants ainsi que de telles touches de fonction sont reproduits graphiquement sur l'écran, sur lequel l'interface utilisateur est visualisée.

24. Installation selon la revendication 23, **caractérisée en ce que** les éléments de réglage de plusieurs appareils sont visualisés en même temps sur l'écran.

25. Installation selon la revendication 23 ou 24, **caractérisée en ce que** seule une sélection configurable par l'opérateur est visualisée en même temps par des éléments de réglage de l'appareil ou des appareils.

26. Installation selon la revendication 25, **caractérisée en ce que** seuls certains paramètres des appareils raccordés au bus peuvent être modifiés en fonction du réglage par défaut appelé.

27. Installation selon l'une quelconque des revendications 16 à 26, **caractérisée en ce que** la commande du calculateur de commande s'effectue au moyen d'une souris, d'une manette ou d'une boule roulante.

28. Installation selon l'une quelconque des revendications 16 à 26, **caractérisée en ce que** la commande du calculateur de commande s'effectue au moyen d'un écran tactile.

29. Installation selon la revendication 27 ou 28, **caractérisée en ce que** les éléments de commande sont réalisés de telle manière qu'une commande est possible en zone stérile.

30. Installation selon l'une quelconque des revendications 1 à 29, **caractérisée en ce que** les fonctions contrôlables via le bus sont commandées par la voix grâce à un module d'entrée et de traitement vocal.

31. Installation selon la revendication 30, **caractérisée en ce que** les commandes vocales sont affichées graphiquement sur un écran ou actionnées acoustiquement.

32. Installation selon la revendication 31, **caractérisée en ce que** des commandes ou des processus de commande entrés de manière incorrecte peuvent être annulés immédiatement.

33. Installation selon l'une quelconque des revendications 1 à 32, **caractérisée en ce qu'**une unité de traitement de signaux vidéo est disponible au niveau de laquelle le signal d'image d'une caméra vidéo est appliqué.

34. Installation selon la revendication 33, **caractérisée en ce que** la caméra vidéo est une caméra vidéo analogue ou numérique.

35. Installation selon la revendication 33 ou 34, **caractérisée en ce que** l'unité de traitement de signaux vidéo présente une unité de traitement d'image.

36. Installation selon l'une quelconque des revendications 33 à 35, **caractérisée en ce que** l'unité de traitement de signaux vidéo numérise l'image enregistrée par la caméra vidéo et la visualise dans une fenêtre de l'interface utilisateur graphique du calculateur de commande.

37. Installation selon l'une quelconque des revendications 33 à 36, **caractérisée en ce que** l'unité de traitement de signaux vidéo numérise l'image enregistrée par la caméra vidéo et la classe dans un calculateur de commande dans lequel des données de base de patients ainsi que des paramètres d'appareil peuvent également être mémorisés en affectation aux images enregistrées.

38. Installation selon l'une quelconque des revendications 33 à 37, **caractérisée en ce que** l'unité de traitement de signaux vidéo visualise l'image enregistrée par la caméra vidéo sur un moniteur analogique ou sur le moniteur du calculateur de commande.

39. Installation selon l'une quelconque des revendications 33 à 38, **caractérisée en ce que** l'unité de traitement de signaux vidéo visualise les informations reçues via le bus dans une superposition vidéo sur le moniteur analogique.

40. Installation selon la revendication 39, **caractérisée en ce que** la superposition vidéo est configurable par l'utilisateur.

41. Installation selon la revendication 40, **caractérisée en ce que** la superposition vidéo affiche des paramètres d'appareil et/ou de traitement et/ou des messages d'erreur.

42. Installation selon l'une quelconque des revendications 33 à 41, **caractérisée en ce que** l'unité de traitement de signaux vidéo présente un microprocesseur qui commande le traitement de signaux vidéo ainsi que, le cas échéant, un processeur graphique qui insère des données et/ou l'image de superposition dans l'image vidéo actuellement enregistrée.

43. Installation selon l'une quelconque des revendications 1 à 42, **caractérisée en ce que** les modifications de réglage effectuées via la commande à distance sont affichées graphiquement sur l'écran du calculateur de commande ou au moyen d'une superposition vidéo.

44. Installation selon l'une quelconque des revendications 1 à 43, **caractérisée en ce que** la caméra vidéo et le moniteur vidéo sont raccordés directement et non via le bus à l'unité de traitement de signaux vidéo.

45. Installation selon l'une quelconque des revendications 1 à 44, **caractérisée en ce qu'**au moins deux appareils sont reliés via le bus en un circuit de réglage fermé.

46. Installation selon la revendication 45, **caractérisée en ce que** le circuit de réglage connecté via le bus interfère avec des réglages internes aux appareils.

47. Installation selon la revendication 46, **caractérisée en ce que** le circuit de réglage connecté via le bus dispose d'une constante de temps sensiblement plus longue que celle de la régulation interne aux appareils.

48. Installation selon l'une quelconque des revendications 45 à 47, **caractérisée en ce que** les appareils reliés via le bus en un circuit de réglage fermé sont
- une installation d'insufflation et une installation d'aspiration
- une source de lumière et une caméra vidéo, et/ou
- une unité de mesure de pression et une pompe ou un insufflateur.

49. Installation selon l'une quelconque des revendications 1 à 48, **caractérisée en ce que** la carte du bus échange des données avec le calculateur de commande via une interface série, un FIFO ou une RAM à double accès.

50. Installation selon la revendication 49, **caractérisée en ce qu'**une fonction de surveillance est prévue qui déclenche une alarme en cas de panne du calculateur de commande ou de la carte du bus.

51. Installation selon l'une quelconque des revendications 1 à 50, **caractérisée en ce que** le logiciel est conçu de manière à ce qu'une panne du logiciel ou du matériel soit indiquée de manière sûre à l'utilisateur de façon qu'un système d'exploitation standard et du matériel standard puissent être utilisés sans perte de sécurité.

52. Installation selon l'une quelconque des revendications 1 à 51, **caractérisée en ce que** le logiciel est conçu de manière à ce que l'utilisateur de l'installation soit clairement identifiable.

53. Installation selon la revendication 52, **caractérisée en ce que** l'entrée de l'udlisateur respectif modifie le réglage de base de l'installation.

54. Installation selon l'une quelconque des revendications 1 à 53, **caractérisée en ce que** le logiciel est conçu de manière à ce que chaque appareil raccordé au bus puisse être configuré individuellement.

55. Installation selon l'une quelconque des revendications 1 à 54, **caractérisée en ce que** le logiciel est conçu de manière à ce que le réglage par défaut des appareils raccordés au bus nécessaire à un certain processus puisse être mémorisé.
